# EUROPEAN PATENT SPECIFICATION

(11) **EP 1 604 660 B1**
(45) Date of publication and mention of the grant of the patent: **05.11.2008**
(21) Application number: 04705942.3
(22) Date of filing: 28.01.2004
(51) Int. Cl.: A61K 31/235, A61K 31/353, A61K 31/43, A61K 31/431, A61K 31/545, A61K 31/546, A61P 31/04

(54) **MEDICINAL COMPOSITION FOR TREATING INFECTION WITH DRUG-RESISTANT STAPHYLOCOCCUS AUREUS**
MEDIZINISCHE ZUSAMMENSETZUNG ZUR BEHANDLUNG VON INFEKTIONEN MIT ARZNEIMITTELRESISTENTEM STAPHYLOCOCCUS AUREUS
COMPOSITION MEDICINALE POUR TRAITER UNE INFECTION DUE AU STAPHYLOCOCCUS AUREUS RESISTANT AUX MEDICAMENTS

(30) Priority: 29.01.2003 JP 2003020611
(43) Date of publication of application: 14.12.2005
(73) Proprietor: MicroBiotech Inc., Naruto-shi, Tokushima 772-0051 (JP)
(72) Inventor: HIGUCHI, Tomihiko, Naruto-shi, Tokushima 772-0051 (JP); SHIBATA, Hirofumi, Itano-gun, Tokushima 771-1262 (JP); SATO, Yoichi, Tokushima-shi, Tokushima 770-0002 (JP); TAKAISHI, Nobuhisa, Tokushima-shi, Tokushima 770-0003 (JP); KAWAZOE, Kazuyoshi, Naruto-shi, Tokushima 779-0225 (JP); MURAKAMI, Kotaro, Kumamoto-shi, Kumamoto 861-5523 (JP)
(74) Representative: Hayes, Adrian Chetwynd
(86) International application number: PCT/JP2004/000751
(87) International publication number: WO 2004/066992

(56) References cited:
- EP-A1- 0 761 226
- EP-A2- 0 443 090
- WO-A1-99/11290
- WO-A2-01/30299
- JP-A- 8 026 991
- JP-A- 10 045 528
- JP-A- 2001 072 596
- DATABASE CHEMABS CHEMICAL ABSTRACTS SERVICE, COLUMBUS, OHIO, US; 2001, DYACHENKO S S ET AL: "The synergistic action of gallic acid with penicillin" XP002413286 retrieved from CAPLUS Database accession no. 1955:54002 & MIKROBIOL. ZHUR. AKAD. NAUK. UKR. R.S.R., vol. 16, no. 2, 1954, pages 47-53,
- DATABASE CHEMABS CHEMICAL ABSTRACTS SERVICE, COLUMBUS, OHIO, US; 2001, DYAXCHENKO S S ET AL: "The synergism of action of gallic acid and penicillin II. Some conditions of the stabilisation of penicillin by the aid of sodium gallate" XP002413287 retrieved from CAPLUS Database accession no. 1958:99972 & MIKROBIOL. ZHUR. AKAD. NAUK. UKR. R.S.R., vol. 20, no. 1, 1958, pages 30-34,
- KUBO ISAO ET AL: "Anti-MRSA activity of alkyl gallates" BIOORGANIC & MEDICINAL CHEMISTRY LETTERS, OXFORD, GB, vol. 12, no. 2, 21 January 2002 (2002-01-21), pages 113-116, XP002246759 ISSN: 0960-894X

## Description

### TECHNICAL FIELD

The invention relates to a phenol derivative for the therapy of an infection with MRSA wherein a characteristic of phenol derivatives which enhance the activity of β-lactam antibiotics on the drug resistant bacteria is utilized. Furthermore, the invention relates to a disinfectant or a functional food having antibacterial activity on MRSA.

### BACKGROUND OF THE INVENTION

Penicillin, which is the first antibiotic, has a β-lactam ring, and has exerted an excellent efficacy toward *Staphylococci.* However, penicillin resistant bacteria which produce an enzyme, penicillinase (β-lactamase), that degrades penicillin has emerged.

In regard to these penicillin resistant bacteria, almost all problems have appeared to be solved in clinical aspects by research and development of penicillinase resistant penicillin such as methicillin and cephems antibiotics. But, a year after methicillin was developed, a strain of MRSA emerged which is resistant to it, and then MRSA which is resistant to all of the antibiotics is clinically isolated.

MRSA have resulted in critical social problems as multiple drug resistant *Staphylococcus aureus* having broad resistance to not only penicillin antibiotics but also cephem antibiotics and aminoglycoside, macrolide, and new quinolone antibiotics.

At present, vancomycin(VCM) etc. is used as antibiotics for MRSA infections, however, short term bactericidal action of VCM is not so potent and VCM is involved in problems of serious side effects such as auricular toxicity and renal toxicity. Therefore, the development of novel antibacterial drugs which are effective on such resistant bacteria is required as an urgent matter.

Dyanchenko S S et al: "The synergistic action of gallic acid with penicillin", Mikrobiol. Zhur. Akad. Nauk. Ukr. R.S.R., vol. 16, no. 2, 1954, pages 47-53, discloses that gallic acid or its salts enhance the antibacterial activity of penicillin.

Dyanchenko S S et al: "The synergism of action of gallic acid and penicillin II. Some conditions of the stabilisation of penicillin by the aid of sodium gallate", Mikrobiol. Zhur. Akad. Nauk. Ukr. R.S.R., vol. 20, no. 1, 1958, pages 30-34, discloses that sodium gallate diminishes the oxidation of penicillin solutions.

### DISCLOSURE OF THE INVENTION

While searching compounds which have anti-MRSA activities among Chinese herbal medicine exhibiting no or weak side effects, the inventors found an interesting fact that extract of "Tara" (*Caesalpinia spinosa*) and multivalent phenol derivatives obtained from the extract suppress the resistance against β-lactam agents, and induce the sensitivity. The present invention was accomplished on the basis of such findings.

The "Tara" in the invention is *Caesalpinia spinosa, leguminosae*, a native of Peru, which is different from "Taranoki" in Japan, and is known to contain ellagic acid which prevents spots and freckles due to sunburn (e.g., LION Life Information, "How to prevent spots and freckles due to sunburn ", Internet, searched on November 6, 1992, <http://www.tion.co.jp/tife/tife3p2.htm>). Also, it is described that gallotannin extracted from "Tara" showed deodorization activity (e.g., Kokai publication No.09-327504).

However, it is not known that extract from "Tara" have enhancing activities on the resistant bacteria by combinations of β-lactam antibiotics.

First aspect of the invention is a phenol derivative of formula V' or its pharmaceutically acceptable salt, for use as an enhancer or β-lactam antibiotics for the treatment of MRSA infections.

In the invention, "Tara" means *Caesalpinia spinosa, leguminosae,* a native of Peru, containing about 0.25% of multivalent phenol derivatives in the whole plant. Extracts from "Tara" means products extracted with a suitable organic solvent or water.

An organic solvent is e.g., methanol or ethanol, and their mixture with water is allowable. Usually, 50% ethanol extract from "Tara" contains about 0.32% of multivalent phenol derivatives. Various gallates from commercial sources can be used in the invention.

Preferable β-lactam antibiotics are oxacillin, cefapirin, ampicillin, penicillin, and cefoxitin; oxacillin, and cefapirin are more preferable.

The phenol derivative of the current invention is a phenol derivative of formula V' 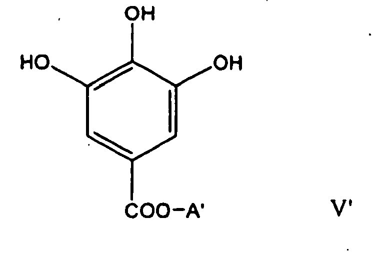 wherein A' is a lower alkyl, and e.g., methyl gallate, ethyl gallate, n-propyl gallate, n-butyl gallate, n-pentyl gallate, n-hexyl gallate, n-heptyl gallate, n-octyl gallate, n-nonyl gallate, n-decyl gallate, n-undecyl gallate, n-lauryl gallate, isobutyl gallate, and isoamyl gallate are included. More preferably, n-propyl gallate, n-pentyl gallate, and isoamyl gallate are included.

Second aspect of the invention is the phenol derivative of the first aspect wherein the lower alkyl group is selected from the group of methyl, ethyl, n-propyl, n-butyl, isobutyl, n-pentyl, isoamyl, hexyl, heptyl, octyl, nonyl, decyl, undecyl and dodecyl.

Third aspect of the invention is the phenol derivative of the first or the second aspect wherein the enhancer is used for the treatment of MRSA infections in combination with a β-lactam antibiotic.

Fourth aspect of the invention is the phenol derivative of the first or the second aspect wherein the enhancer is used in a disinfectant for MRSA in combination with a β-lactam antibiotic.

Fifth aspect of the invention is the phenol derivative of the first or the second aspect wherein the enhancer is used in a functional food having antibacterial activity for MRSA in combination with a β-lactam antibiotic.

Sixth aspect of the invention is the use of a phenol derivative of formula V', wherein A' is a lower alkyl group having I to 12 carbon atoms, or a pharmaceutically acceptable salt thereof, for use as an enhancer of β-lactam antibiotics for the treatment of MRSA infections.

Seventh aspect of the invention is a pharmaceutical composition for the therapy of an infection with MRSA comprising a β-lactam antibiotic and a phenol derivative of formula V', or a pharmaceutically acceptable salt thereof.

Eighth aspect of the invention is a disinfectant having antibacterial activity on MRSA comprising a β-lactam antibiotic and a phenol derivative of formula V' or a pharmaceutically acceptable salt thereof as an active ingredient.

Ninth aspect of the invention is a functional food for the prevention and/or improvement of an infection with MRSA comprising a phenol derivative of formula V', or a pharmaceutically acceptable salt thereof, which enhance antibacterial activity of a β-lactam antibiotic on the resistant bacteria.

### BEST MODE FOR CARRYING OUT THE INVENTION

The term "Lower alkyl" in the invention refers to saturated straight or branched hydrocarbon chain having 1 to 12 carbon atoms. For example, methyl, ethyl, n-propyl, n-butyl, n-pentyl, n-hexyl, n-heptyl, n-octyl, n-nonyl, n-decyl, n-undecyl, n-lauryl, isobutyl, and isoamyl are included.

Phenol derivatives, antibiotics and antibacterial agents in the invention include pharmaceutically acceptable salts of them. Pharmaceutically acceptable salts refer to medicinally allowable salts commonly used, e.g., salts of sodium, potassium or calcium, or acid-additive salts such as amine salts(e.g., a dibenzylamine salt) and HCl salt. Also, other active ingredients may be included in the invention.

Examples of the β-lactam antibiotics used in the invention include benzylpenicillin, phenoxymethylpenicillin, phenethicillin, propicillin, ampicillin, methicillin, oxacillin, cloxacillin, flucloxacillin, dicloxacillin, hetacillin, talampicillin, bacampicillin, lenampicillin, amoxicillin, ciclacillin, carbenicillin, sulbenicillin, ticarcillin, carindacillin, carfecillin, piperacillin, mezlocillin, aspoxicillin, cephaloridine, cefazolin, cefapirin, cephacetrile, ceftezole, cephaloglycin, cephalexin, cefatrizine, cefaclor, cefroxadine, cefadroxil, cefamandole, cefotiam, cephalothin, cephradine, cefuroxime, cefoxitin, cefotaxime, ceftizoxime, cefmenoxime, cefodizime, ceftriaxone, cefuzonam, ceftazidime, cefepim, cefpirome, cefozopran, cefoselis, cefluprenam, cefoperazone, cefpimizole, cefpiramide, cefixime, cefteram pivoxil, cefpodoxime proxetil, ceftibuten, cefetamet pivoxil, cefdinir, cefditoren pivoxil, cefcapene pivoxil, cefsulodin, cefoxitin, cefmetazole, latamoxef, cefotetan, cefbuperazone, cefminox, flomoxef, aztreonam, carumonam, imipenem, panipenem, meropenem, viapenem, faropenem, ritipenem acoxil, or mixtures thereof. The β-lactam antibiotics are preferably ampicillin, benzylpenicillin, phenethicillin, methicillin, oxacillin, carbenicillin, cefapirin, cefradine, cefuroxime, cefoxitin, cefotaxime, and panipenem; more preferably, ampicillin, cefapirin, benzylpenicillin, oxacillin, cefoxitin or mixtures thereof.

The antibiotics may be in the form of a pharmaceutically acceptable salt. Pharmaceutically acceptable salts refer to salts which are allowable in medical aspects used in general as salts of an antibiotic, including for example, salts of sodium, potassium, calcium and the like, and amine salts of procaine, dibenzylamine, ethylenediamine, ethanolamine, methylglucamine, taurine, and the like, as well as acid addition salts such as hydrochlorides, and basic amino acids and the like.

Mode of administration of the phenol derivatives of formula V' includes parenteral administration, oral administration or topical administration similarly to the case of conventional antibiotics. In general, the administration in an injectable is suitable. In this instance, the injectable is prepared by the conventional process, which also involves the cases in which the compound is dissolved in an adequate vehicle, e.g., sterilized distilled water, saline and the like, to give an injectable form.

Moreover, the phenol derivatives of formula V' can be orally administered by the combination with a β-lactam antibiotics in a variety of dosage forms. Examples of the dosage form include for example, tablet, capsule, sugarcoated tablet or the like, liquid solution or suspension.

Total dose of both agents of the phenol derivative of formula V' and the β-lactam antibiotic may vary depending on types of the combined agent, the ratio of the combined use, or the age, body weight, symptoms of the patient and the route for administration. For example, when administered to an adult (body weight: about 50 kg), 10 mg - 2 g in total weight of both agents combined per single dosage is administered from once to three times per a day. To achieve the best therapeutic effects may be intended by altering the dose and route for administration.

In accordance with the invention, it is possible to apply a wide range of weight ratio of the phenol derivative of formula V' and the β-lactam antibiotic which are used in combination or admixed together. Further, because the ratio of the combined use varies depending on types and severity of the infection, and types of the β-lactam antibiotic used in combination, the ratio of the combined use is not particularly limited. Accordingly, combination of the concentration by which effects through the combined use can be expected is achieved upon combination in the range of usual dosages.

The pharmaceutical composition of the invention is usually prepared according to the conventional process, and is administered in a pharmaceutically adequate form. For example, solid peroral form may include a diluent such as lactose, dextrose, saccharose, cellulose, and cornstarch and potato starch, a lubricant such as silica, talc, stearic acid, magnesium stearate or calcium stearate, and/or polyethyleneglycol, a binder such as starch, gum arabic, gelatin, methyl cellulose, carboxymethylcellulose, polyvinyl pyrrolidine, a disintegrant such as starch, alginic acid, alginate, glycolic acid starch sodium, a foaming agent, a pigment, an edulcorant, a wetting agent such as lecithin, polysorbate, lauryl sulfate, and a pharmaceutically inactive substance which is generally nontoxic and used for a pharmaceutical formulation, in addition to the active compound.

The above-described pharmaceutical composition is produced by a known process such as for example, mixing, granulation, and manufacture of tablets, sugar coating, or coating process.

In a case of parenteral administration, suppository to which rectal application is intended is also feasible, however, frequently used dosage form is an injectable. The injectable includes dosage forms having different appearances such as liquid formulations, formulations for dissolution before use, and suspension formulations, which are fundamentally identical in respect of requiring sterilization of the active ingredient by an appropriate method, followed by directly placing into a vessel, and sealing.

Most convenient formulation process includes a process in which the active ingredient is sterilized by an appropriate method, thereafter separately, or after being physically mixed, the aliquot thereof is separately formulated. Further, when a liquid dosage form is selected, a process can be applied in which the active ingredient is dissolved in an appropriate medium, followed by sterilization by filtration, filling in an appropriate ampoule or vial, and sealing.

In this case, frequently used media include distilled water for injection, which is not limited thereto in accordance with the invention. Additionally, additives such as soothing agents having a local anesthetic action such as procaine hydrochloride, xylocaine hydrochloride, benzyl alcohol and phenol, antiseptic agents such as benzyl alcohol, phenol, methyl or propylparaben and chlorobutanol, buffer agents such as a sodium salt of citric acid, acetic acid, phosphoric acid, auxiliary agents for the dissolution such as ethanol, propylene glycol, arginine hydrochloride, stabilizing agents such as L-cysteine, L-methionine, L- histidine, as well as isotonizing agents can be also added, if required.

The phenol derivative of formula V' can be prepared as an antibacterial agent or a bactericidal agent. The antibacterial agent or the bactericidal agent is comprising about 0.1- 10% in weight of phenol derivative of formula V', and a suitable amount of β-lactam antibiotics. These antibacterial agents or bactericidal agents are used to disinfect instruments such as scissors, scalpels, catheters, as well as excrements of patients, and to irrigate skins, mucosa and wounds.

The enhancer of the invention can be applied as a form of a functional food to prevent an infection with a drug resistant bacterium.

A form of the invention when used as a food is not limited and for example, a drink, a solid product, and jellied food are included; in the solid product a processed form as a preparation of powder, granule, tablet and capsule is included. The phenol derivative of formula V' may be contained in a noodle like udon (a wheat noodle) or soba(a buckwheat noodle), a cookie, a biscuit, a candy, bread, a cake, and other foods; it may be added in drinks such as a carbonated drink, lactic acid drink etc.

### TEST EXAMPLES

### Test Example 1 Enhancing activity for the antibacterial activity of oxacillin by various gallates (by the agar plate dilution method)

According to the agar plate dilution method defined by Japan Society of Chemotherapy, MIC (Minimum inhibitory concentration) was determined. CAMHA was prepared by adding 50 mg/l of Ca-ion, 25mg/l of Mg-ion, and 2% of NaCl to Mueller-Hinton II Agar (MHA), and it was used as a medium for use in the measurement of sensitivity. The bacterial liquid was incubated at 37°C overnight and diluted to 10⁶ CFU/ml with saline. To the plate for use in the measurement of sensitivity was seeded the bacterial liquid with a micro planter (Sakuma Seisakusho), and MIC was determined after incubation at 37°C for 24 hr. The results were shown in Table 2-1 and 2-2.

**Table 2-1**

| Oxacillin MIC (µ g/ml) | | | | | | | | | | | | | | | |
|---|---|---|---|---|---|---|---|---|---|---|---|---|---|---|---|
| Strain No. | - | Methyl gallate | | Ethyl gallate | | Propyl gallate | | Butyl gallate | | Pentyl gallate | | Hexyl gallate | | Heptyl gallate | |
| | | 25µg/ml | 50µg/ml | 25µg/ml | 50µg/ml | 25µg/ml | 50µg/ml | 25µg/ml | 50µg/ml | 12.5µg/ml | 25µg/ml | 12.5µg/ml | 25µg/ml | 12.5µg/ml | 25µg/ml |
| MRSA 1 | >256 | >256 | >256 | 256 | >256 | 256 | 128 | 128 | 16 | 258 | 64 | >256 | 128 | 256 | >256 |
| 2 | 256 | 64 | 8 | 4 | 0.25 | 2 | 0.063 | 4 | ND | 8 | 0.063 | 64 | 0.031 | 8 | ND |
| 3 | 128 | 32 | 8 | 8 | 8 | 8 | 2 | 4 | 0.5 | 8 | 0.25 | 64 | 2 | 64 | 2 |
| 4 | 256 | 256 | 64 | 128 | <0.016 | 128 | <0.016 | 64 | ND | 128 | 2 | >256 | 16 | 256 | ND |
| 5 | 256 | 64 | 16 | 16 | 8 | 8 | 1 | 8 | <0.016 | 64 | 2 | 64 | 4 | 128 | 32 |
| 6 | >256 | >256 | 25 | 256 | 1 | 256 | <0.016 | 64 | ND | 32 | 1 | 256 | 2 | 256 | ND |
| 7 | >256 | 128 | 16 | 32 | 8 | 32 | 2 | 16 | <0.016 | 32 | 0.13 | 256 | 2 | 256 | 32 |
| 8 | 128 | 128 | 64 | 32 | 16 | 16 | 2 | 16 | ND | 16 | 0.13 | 64 | 2 | 128 | 32 |
| 9 | 256 | 128 | ND | 16 | ND | 8 | ND | 16 | <0.016 | 128 | 0.25 | >256 | 2 | 256 | ND |
| 10 | 128 | 2 | 2 | 1 | <0.016 | 0.5 | ND | <0.5 | ND | 4 | 0.13 | ND | ND | 0.5 | 0.25 |
| 12 | >256 | 256 | 256 | 64 | 256 | 16 | 16 | 64 | 8 | 128 | 16 | 256 | 16 | 128 | 256 |
| 16 | 256 | 256 | 256 | 128 | 16 | 128 | 2 | 128 | ND | 128 | 2 | 256 | 4 | 256 | 64 |
| 17 | 256 | 128 | 4 | 64 | <0.016 | 32 | 1 | 32 | 0.25 | 64 | 0.25 | 256 | 4 | 128 | 2 |
| 18 | >256 | 256 | 32 | 128 | 0.13 | 128 | 0.125 | 64 | <0.016 | 128 | 2 | 256 | 4 | 256 | 2 |
| 20 | 64 | 4 | 8 | 2 | 4 | 2 | 1 | 1 | 0.5 | 1 | 0.5 | 8 | 1 | 8 | 2 |
| 21 | 64 | 16 | 8 | 4 | 8 | 2 | 2 | 2 | 1 | 16 | 2 | 8 | 4 | 16 | 32 |
| 22 | 32 | 16 | 16 | 8 | 8 | 2 | 4 | 2 | 2 | 8 | 1 | 1 | 4 | 16 | 32 |
| 24 | <0.5 | <0.5 | 1 | <0.5 | 0.5 | 0.5 | 0.5 | <0.5 | 0.25 | 0.5 | 0.25 | 1 | 1 | 0.5 | 0.13 |
| COL | 256 | 128 | 84 | 84 | 32 | 32 | 8 | 32 | 4 | 32 | 2 | 256 | 32 | 64 | 32 |
| MSSA 1003 | 0.25 | <0.5 | 1 | 1 | 0.5 | 0.5 | 0.5 | <0.5 | 0.5 | 0.25 | 0.031 | 0.25 | 0.031 | 0.13 | ND |
| 1010 | 0.25 | 1 | 2 | 1 | 1 | 1 | 1 | 1 | 0.5 | 0.25 | 0.25 | 0.5 | 0.031 | 0.25 | ND |
| 1020 | 1 | <0.5 | 1 | 1 | 1 | 0.25 | 0.25 | <0.5 | 0.13 | 0.5 | 0.25 | 1 | 0.031 | 0.5 | ND |
| 1023 | 2 | 1 | 1 | 1 | 1 | 1 | 1 | <0.5 | 0.5 | 1 | 0.5 | 1 | 1 | 0.5 | 0.25 |
| 1029 | 4 | 1 | 1 | 1 | 1 | 1 | 1 | 1 | 1 | 2 | 1 | 2 | 1 | 0.5 | 0.25 |
| 1032 | 0.13 | <0.5 | 1 | 1 | 1 | 0.5 | 0.5 | <0.5 | 0.25 | 0.25 | 0.25 | 0.5 | 0.13 | 0.25 | 0.25 |
| ATCC 6538 | 0.063 | <0.5 | 0.25 | <0.5 | 0.25 | 0.13 | 0.25 | <0.5 | 0.13 | 0.13 | 0.13 | 0.25 | 0.031 | 0.063 | ND |
| RN4220 | 0.13 | <0.5 | 0.25 | <0.5 | 0.25 | 0.25 | 0.125 | <0.5 | ND | 0.13 | 0.063 | 0.13 | 0.13 | 0.063 | ND |

| | | | | | | | | | | | | | | | |
|---|---|---|---|---|---|---|---|---|---|---|---|---|---|---|---|
| ND : not determine | | | | | | | | | | | | | | | |

**Table 2-2**

| Oxacillin MIC (µg/ml) | | | | | | | | | | | | | | |
|---|---|---|---|---|---|---|---|---|---|---|---|---|---|---|
| Strain No. | Octyl gallate | | Nonyl gallate | | Decyl gallate | | Undecyl gallate | | Lauryl gallate | | isoButyl gallate | | isoAmyl gallate | |
| | 12.5µg/ml | 25µg/ml | 12.5µg/ml | 25µg/ml | 12.5µg/ml | 25µg/ml | 12.5µg/ml | 25µg/ml | 12.5µg/ml | 25µg/ml | 25µg/ml | 50µg/ml | 12.5µg/ml | 25µg/ml |
| MRSA 1 | 256 | ND | 256 | ND | >256 | ND | >256 | ND | 256 | >256 | 256 | 32 | >256 | 64 |
| 2 | 0.13 | ND | 0.13 | ND | 32 | ND | 16 | ND | 256 | >256 | 8 | ND | 128 | 0.5 |
| 3 | 8 | ND | 128 | ND | 128 | ND | 256 | ND | 128 | 256 | 4 | 0.5 | 128 | 0.063 |
| 4 | 2 | ND | 2 | ND | 16 | ND | >256 | ND | 256 | 256 | 128 | 1 | 256 | 8 |
| 5 | 64 | ND | 0.13 | ND | 1 | ND | 256 | ND | 256 | 256 | 16 | '0.25 | 128 | 0.063 |
| 6 | 64 | ND | 0.063 | ND | 0.063 | ND | >256 | ND | 256 | >256 | 128 | ND | 128 | 1 |
| 7 | 64 | ND | 128 | ND | 128 | ND | >256 | ND | 256 | >256 | 16 | ND | 256 | <0.063 |
| 8 | 128 | ND | 256 | ND | 256 | ND | >256 | ND | 128 | 256 | 16 | 1 | 256 | 0.063 |
| 9 | 1 | ND | 0.5 | ND | 128 | ND | 256 | ND | 256 | 256 | 128 | ND | 256 | 4 |
| 10 | 32 | ND | ND | ND | ND | ND | ND | ND | 8 | 4 | <0.5 | ND | 32 | ND |
| 12 | 128 | ND | 256 | ND | 256 | ND | 256 | ND | 256 | >256 | 128 | 8 | 256 | 4 |
| 16 | 128 | ND | 1 | ND | 2 | ND | 256 | ND | 256 | 256 | 256 | 1 | 256 | 4 |
| 17 | 1 | ND | 0.5 | ND | 2 | ND | 256 | ND | 256 | 256 | 128 | 1 | 128 | 4 |
| 18 | 4 | ND | 0.13 | ND | 16 | ND | >256 | ND | 256 | >256 | 128 | 1 | 256 | 16 |
| 20 | 16 | ND | 4 | ND | 4 | ND | 8 | ND | 8 | 64 | 1 | 0.5 | 4 | 0.25 |
| 21 | 16 | ND | 32 | ND | 16 | ND | 32 | ND | 32 | 64 | 4 | 1 | 32 | 0.5 |
| 22 | 64 | ND | 32 | ND | 64 | ND | 128 | ND | 32 | 128 | 4 | 2 | 32 | 1 |
| 24 | 0.13 | ND | 0.063 | ND | 0.063 | ND | 0.5 | ND | <0.5 | 0.5 | 1 | 0.25 | 1 | 0.5 |
| COL | 128 | ND | 0.063 | ND | 0.13 | ND | 256 | ND | 256 | 256 | 64 | 4 | 128 | 2 |
| MSSA 1003 | 0.13 | ND | 0.063 | ND | 0.13 | ND | 0.063 | ND | <0.5 | 0.13 | <0.5 | 0.5 | 0.25 | 0.25 |
| 1010 | 0.13 | ND | 0.13 | ND | 0.13 | ND | 0.25 | ND | <0.5 | 2 | 1 | 0.5 | 0.25 | 0.25 |
| 1020 | 0.13 | ND | 0.063 | ND | 0.063 | ND | 0.25 | ND | 1 | 2 | <0.5 | 0.13 | 1 | 0.25 |
| 1023 | 0.13 | ND | 0.063 | ND | 0.063 | ND | 0.5 | ND | 2 | 2 | 1 | 0.5 | 2 | 0.25 |
| 1029 | 0.13 | ND | 0.063 | ND | 0.13 | ND | 0.5 | ND | 1 | 0.5 | 1 | 0.5 | 4 | 0.5 |
| 1032 | 0.13 | ND | 0.063 | ND | 0.063 | ND | 0.13 | ND | <0.5 | 0.5 | <0.5 | 0.25 | 0.25 | 0.25 |
| ATCC 6538 | 0.13 | ND | 0.063 | ND | 0.063 | ND | 0.13 | ND | <0.5 | 0.13 | <0.5 | 0.25 | 0.13 | 0.13 |
| RN4220 | 0.063 | ND | 0.063 | ND | 0.063 | ND | 0.13 | ND | <0.5 | 0.13 | <0.5 | 0.063 | 0.13 | 0.063 |

| | | | | | | | | | | | | | | |
|---|---|---|---|---|---|---|---|---|---|---|---|---|---|---|
| ND : not determine | | | | | | | | | | | | | | |

### Result

With respect to the effect of combination use with oxacillin, the effect was potentiated as the number of carbon atoms in the side chain of the multivalent phenol was increased, and MIC for oxacillin with MRSA No.2 was elevated to 0.06 µg/ml from 256 µg/ml by 25 µg/ml of n-pentyl gallate. But the effect of the combination use was decreased when the number of carbon atom was further increased, and C4-C6 of the side chain was found to show the most potential effect of the combination use. These effects were found in MSSA as well as in MRSA. Furthermore, isobutyl gallate and isoamyl gallate, the side chains of which were branched, also have the potent effects of the combination use.

In addition, MIC for oxacillin alone with MRSA Strain No. 2 is 256 µg/ml, MIC for n-pentyl gallate alone is 67.5 µg/ml as shown in Table 3-1 below; however, MIC for oxacillin was elevated to 0.063 µg/ml when combined with 25 µg/ml of n-pentyl gallate showing no antibacterial effect, and n-pentyl gallate is found to have excellent potentiating effect. Test Example 2 Antibacterial activity of gallic acid and various gallates alone (by the agar plate dilution method)

MIC for gallic acid, various gallates and multivalent phenol derivatives alone with MRSA and MSSA were determined as described in experiment 2. The results were shown in Table 3-1 and 3-2.

**Table 3-1**

| Name | Gallic acid | Methyl gallate | Ethyl gallate | Propyl gallate | Butyl gallate | Pentyl gallate | Hexyl gallate | Heptyl gallate | Octyl gallate | Nonyl gallate | Decyl gallate |
|---|---|---|---|---|---|---|---|---|---|---|---|
| MRSA #1 | 31.3 | 250 | 125 | 125 | 125 | 62.5 | 62.5 | 31.3 | 31.3 | 15.7 | 15.7 |
| MRSA #2 | 62.5 | 125 | 62.5 | 62.5 | 125 | 62.5 | 31.3 | 31.3 | 31.3 | 15.7 | 15.7 |
| MRSA #3 | 31.3 | 250 | 125 | 125 | 125 | 62.5 | 31.3 | 31.3 | 31.3 | 15.7 | 15.7 |
| MRSA #4 | 62.6 | 125 | 82.5 | 125 | 125 | 62.5 | 31.3 | 31.3 | 31.3 | 15.7 | 15.7 |
| MRSA #5 | 31.3 | 250 | 125 | 125 | 125 | 62.5 | 31.3 | 31.3 | 31.3 | 15.7 | 15.7 |
| MRSA #6 | 31.3 | 125 | 62.5 | 62.5 | 62.5 | 62.5 | 31.3 | 31.3 | 31.3 | 15.7 | 15.7 |
| MRSA #7 | 62.5 | 250 | 125 | 125 | 125 | 62.5 | 31.3 | 31.3 | 31.3 | 15.7 | 15.7 |
| MRSA #8 | 31.3 | 250 | 125 | 125 | 125 | 62.5 | 31.3 | 31.3 | 31.3 | 15.7 | 15.7 |
| MRSA #9 | 31.3 | 62.5 | 62.5 | 62.5 | 62.5 | 62.5 | 31.3 | 31.3 | 31.3 | 15.7 | 15.7 |
| MRSA #10 | 31.3 | 62.5 | 62.5 | 62.5 | 62.5 | 62.5 | 31.3 | 31.3 | 15.6 | 15.7 | 15.7 |
| MRSA #12 | 62.5 | 250 | 125 | 125 | 125 | 62.5 | 62.5 | 31.3 | 31.3 | 15.7 | 15.7 |
| MRSA #16 | 31.3 | 125 | 125 | 125 | 125 | 62.5 | 62.5 | 31.3 | 31.3 | 15.7 | 15.7 |
| MRSA #11 | 62.5 | 125 | 62.5 | 62.6 | 125 | 62.5 | 62.5 | 31.3 | 31.3 | 15.7 | 15.7 |
| MRSA #18 | 62.5 | 125 | 62.5 | 62.5 | 125 | 62.5 | 62.5 | 31.3 | 31.3 | 15.7 | 15.7 |
| MRSA #20 | 62.5 | 250 | 125 | 125 | 125 | 62.5 | 62.5 | 31.3 | 31.3 | 15.7 | 15.7 |
| MRSA #21 | 62.5 | 250 | 125 | 125 | 125 | 62.5 | 62.5 | 31.3 | 31.3 | 15.7 | 31.3 |
| MRSA #22 | 62.5 | 250 | 125 | 125 | 125 | 62.5 | 62.5 | 31.3 | 31.3 | 15.7 | 31.3 |
| MRSA #24 | 62.5 | 250 | 125 | 125 | 125 | 62.5 | 62.5 | 31.3 | 31.3 | 15.7 | 31.3 |
| COL | 62.5 | 250 | 125 | 125 | 125 | 62.5 | 31.3 | 31.3 | 31.3 | 15.7 | 15.7 |
| MSSA #1003 | 62.5 | 250 | 125 | 125 | 125 | 62.5 | 62.5 | 31.3 | 31.3 | 15.7 | 15.7 |
| MSSA #1010 | 62.5 | 250 | 125 | 125 | 125 | 62.5 | 62.5 | 31.3 | 31.3 | 15.7 | 15.7 |
| MSSA #1020 | 62.5 | 250 | 125 | 125 | 125 | 62.5 | 62.5 | 31.3 | 31.3 | 15.7 | 15.7 |
| MSSA #1023 | 31.3 | 125 | 62.5 | 62.5 | 125 | 62.5 | 62.5 | 31.3 | 31.3 | 15.7 | 15.7 |
| MSSA #1029 | 31.3 | 62.5 | 62.5 | 62.5 | 62.5 | 62.5 | 62.5 | 31.3 | 31.3 | 15.7 | 15.7 |
| -MSSA #1032 | 31.3 | 250 | 125 | 125 | 125 | 62.5 | 62.5 | 31.3 | 31.3 | 15.7 | 15.7 |
| ATCC6538 | 62.5 | 250 | 125 | 125 | 125 | 62.5 | 31.3 | 31.3 | 31.3 | 15.7 | 15.7 |
| RN4220 | 62.5 | 125 | 125 | 62.5 | 62.5 | 62.5 | 62.5 | 31.3 | 31.3 | 15.7 | 15.7 |
| MIC₅₀ | 62.5 | 250 | 125 | 125 | 125 | 62.5 | 62.5 | 31.3 | 31.3 | 15.7 | 15.7 |

| | | | | | | | | | | | |
|---|---|---|---|---|---|---|---|---|---|---|---|
| (µg/ml) | | | | | | | | | | | |

**Table 3-2**

| Name | Undecyl gallate | Lauryl gallate | Cetyl gallate | Isobutyl gallate | Isoamyl gallate | 2',4',5'-trihydroxy butyrophonone | 2', 3',4'-trihyd- roxyacetophenone | Isoamyl 4- hydroxybenzoate | n-Amyl 4- hydroxybenzoate |
|---|---|---|---|---|---|---|---|---|---|
| MRSA #1 | 31.3 | 62.5 | >250 | 125 | | 62.5 | 62.5 | 62.5 | 62.5 |
| MRSA #2 | 31.3 | 62.5 | 250 | 62.5 | 62.5 | 125 | 62.5 | 62.5 | 62.5 |
| MRSA #3 | 31.3 | 62.5 | 250 | 125 | 62.5 | 62.5 | 62.5 | 62.5 | 62.5 |
| MRSA #4 | 31.3 | 62.5 | >250 | 125 | 62.5 | 125 | 62.5 | 62.5 | 62.5 |
| MRSA #5 | 31.3 | 62.5 | >250 | 125 | 62.5 | 62.5 | 62.5 | 62.5 | 62.5 |
| MRSA #6 | 31.3 | 31.3 | 125 | 62.5 | 62.5 | 62.5 | 31.3 | 62.5 | 31.3 |
| MRSA #7 | 31.3 | 31.3 | >250 | 125 | 62.5 | 62.5 | 62.5 | 62.5 | 62.5 |
| MRSA #8 | 31.3 | 31.3 | >250 | 125 | 62.5 | 125 | 62.5 | 62.5 | 31.3 |
| MRSA #9 | 31.3 | 31.3 | >250 | 62.5 | 62.5 | 62.5 | 62.5 | 62.5 | 31.3 |
| MRSA #10 | 31.3 | 15.6 | 125 | 62.5 | 31.3 | 62.5 | 31.3 | 31.3 | 62.5 |
| MRSA #12 | 31.3 | 62.5 | >250 | 125 | 62.5 | 125 | 62.5 | 62.5 | 62.5 |
| MRSA #16 | 31.3 | 62.5 | >250 | 125 | 62.5 | 62.5 | 62.5 | 62.5 | 62.5 |
| MRSA #17 | 31.3 | 62.5 | >250 | 125 | 62.5 | 126 | 62.5 | 62.5 | 62.5 |
| MRSA #18 | 31.3 | 62.5 | >250 | 125 | 62.5 | 125 | 62.5 | 62.5 | 62.5 |
| MRSA #20 | 31.3 | 62.5 | >250 | 125 | 62.5 | 125 | 62.5 | 62.5 | 62.5 |
| MRSA #21 | 31.3 | 62.5 | >250 | 125 | 62.5 | 125 | 62.5 | 62.5 | 62.5 |
| MRSA #22 | 31.3 | 62.5 | >250 | 125 | 125 | 125 | 62.5 | 62.5 | 62.5 |
| MRSA #24 | 31.3 | 62.5 | >250 | 125 | 125 | 125 | 62.5 | 62.5 | 62.5 |
| COL | 31.3 | 31.3 | 250 | 125 | 62.5 | 125 | 62.5 | 62.5 | 62.5 |
| MSSA #1003 | 31.3 | 62.6 | >250 | 125 | 125 | 125 | 62.5 | 62.5 | 62.5 |
| MSSA #1010 | 31.3 | 62.5 | >250 | 125 | 62.5 | 125 | 62.5 | 62.5 | 62.5 |
| MSSA #1020 | 31.3 | 62.5 | >250 | 125 | 62.5 | 126 | 62.5 | 62.5 | 62.5 |
| MSSA #1023 | 31.3 | 62.5 | >250 | 125 | 62.5 | 62.5 | 31.3 | 62.5 | 31.3 |
| MSSA #1029 | 31.3 | 62.5 | 250 | 62.5 | 62.5 | 62.5 | 31.3 | 62.5 | 62.5 |
| MSSA #1032 | 31.3 | 62.5 | 250 | 125 | 62.5 | 62.5 | 62.5 | 62.5 | 62.5 |
| ATCC6538 | 31.3 | 62.5 | >250 | 125 | 62.5 | 125 | 62.5 | 62.5 | 62.5 |
| RM4220 | 31.3 | 62.5 | 250 | 125 | 62.5 | 125 | 62.5 | 62.5 | 62.5 |
| MIC₅₀ | 31.3 | 62.5 | >250 | 125 | 62.5 | 125 | 62.5 | 62.5 | 62.5 |

| | | | | | | | | | |
|---|---|---|---|---|---|---|---|---|---|
| (µg/ml) | | | | | | | | | |

### Result

As shown in Table 3, antibacterial activities of gallic acid and various gallates alone with MRSA and MSSR were potentiated as the number of carbon atoms was increased, and nonyl gallate and decyl gallate in the range of C9-C 10 were most potent and their MICs were 15.7 µg/ml. The activity was, however, decreased if the number of carbon atom was further increased.

### Test Example 3 Potentiating effect of isoamyl gallate for the antibacterial activity of other β-lactams (by the agar plate dilution method)

Since isoamyl gallate showed excellent effect of the combination use, MIC was determined as described in example 2 and the effect of the combination use of isoamyl gallate and other β-lactams was studied.

**Table 4**

| | Penicillin G (mg/ml) | | Cefoxitin (mg/ml) | | Ampicillin (mg/ml) | | Cefapirin(mg/ml) | |
|---|---|---|---|---|---|---|---|---|
| Strain No. | alone | Isoamyl gallate 25mg/ml | alone | Isoamyl gallate 25mg/ml | alone | Isoamyl gallate 25mg/ml | alone | Isoamyl gallate 25mg/ml |
| MRSA 1 | 64 | 8 | >256 | 64 | 32 | 8 | 128 | 16 |
| 2 | 64 | 0.5 | 256 | 4 | 64 | 0.5 | 128 | 0.06 |
| 3 | 32 | 0.25 | 256 | 0.25 | 16 | 1 | 64 | 0.016 |
| 4 | 32 | 4 | >256 | 16 | 32 | 4 | 64 | 4 |
| 5 | 64 | 2 | >256 | 16 | 32 | 2 | 64 | 4 |
| 6 | 32 | 1 | 256 | 4 | 32 | 2 | 64 | 0.5 |
| 7 | 32 | 8 | >256 | 4 | 32 | 4 | 64 | 0.016 |
| 8 | 32 | 0.063 | 256 | 0.5 | 16 | 0.25 | 64 | 0.016 |
| 9 | 32 | 2 | >256 | 4 | 32 | 4 | 64 | 0.5 |
| 10 | 32 | 0.5 | 64 | 4 | 32 | 0.5 | 64 | 0.063 |
| 12 | 32 | 4 | >256 | 32 | 32 | 4 | 128 | 4 |
| 16 | 32 | 8 | >256 | 16 | 32 | 8 | 64 | 4 |
| 17 | 32 | 1 | 256 | 4 | 32 | 1 | 64 | 0.5 |
| 18 | 32 | 4 | >256 | 16 | 32 | 4 | 64 | 4 |
| 20 | 16 | 0.063 | 32 | 2 | 8 | 0.25 | 16 | 0.063 |
| 21 | 16 | 0.25 | 64 | 2 | 8 | 0.5 | 32 | 0.5 |
| 22 | 16 | 0.25 | 64 | 4 | 8 | 0.5 | 16 | 0.5 |
| 24 | 8 | 0.25 | 8 | 0.5 | 16 | 0.5 | 0.25 | 0.063 |
| COL | 32 | 1 | >256 | 16 | 32 | 2 | 64 | 1 |
| MSSA 1003 | 0.13 | 0.063 | 4 | 2 | 0.5 | 0.25 | 0.5 | 0.063 |
| 1010 | 0.13 | 0.063 | 4 | 2 | 0.5 | 0.13 | 0.25 | 0.063 |
| 1020 | 0.13 | 0.031 | 4 | 0.25 | 0.25 | 0.063 | 0.25 | 0.063 |
| 1023 | 4 | 0.25 | 8 | 2 | 8 | 0.5 | 0.5 | 0.063 |
| 1029 | 8 | 0.25 | 8 | 2 | 16 | 0.5 | 0.5 | 0.063 |
| 1032 | 0.063 | 0.063 | 2 | 0.5 | 0.13 | 0.13 | 0.063 | 0.063 |
| ATCC 6538 | 0.063 | <0.016 | 2 | 0.031 | 0.25 | 0.031 | 0.063 | 0.016 |
| RN4220 | 0.031 | 0.031 | 2 | 0.25 | 0.063 | 0.063 | 0.063 | 0.063 |

As shown in Table 4, isoamyl gallate showed excellent effects of the combination use with penicillin G, cefoxitin, ampicillin, and cefapirin.

### Test Example 5 Potentiating effect of various gallates for antibacterial activity of oxacillin [measurement of MIC (by broth microdilution method) and FIC]

According to the broth microdilution method defined by Japan Society of Chemotherapy, MIC was determined. CAMHA was prepared by adding 50 mg/l of Ca-ion, 25mg/l of Mg-ion, and 2% of NaCl to MHB (Mueller-Hinton Broth), and it was used as a medium for use in the measurement of sensitivity. In 96-well microplates 100 µl of the medium for use in the measurement of sensitivity containing drugs was dispensed. The bacterial liquid was incubated at 37°C overnight, diluted with saline and added to the plate so that the final concentration of the bacteria was 5 x 10⁵ CFU/well. After incubation at 37°C for 24 hr, MIC was determined from the presence or absence of the bacterial development. FIC (fractional inhibitory concentration) was calculated from the result and the potency of the effect of the combination use was evaluated. The results were shown in Table 5.

**Table 5**

| Compound | Oxacillin | | | |
|---|---|---|---|---|
| | MRSA COL | | MRSA Strain No.5 | |
| | MIC (µg/ml) | FIC value | MIC (µg/ml) | FIC value |
| Methyl gallate | >1600 | - | >1600 | - |
| Ethyl gallate | 400 | 0.125 | 400 | 0.156 |
| Propyl gallate | 100 | 0.258 | 100 | 0.258 |
| Butyl gallate | 100 | 0.129 | 50 | 0.254 |
| Isobutyl gallate | 50 | 0.129 | 50 | 0.266 |
| Pentyl gallate | 25 | 0.375 | 12.5 | 0.563 |
| Isoamyl gallate | 25 | 0.25 | 25 | 0.313 |
| Hexyl gallate | 6.25 | 0.5 | 6.25 | 0.502 |
| Heptyl gallate | 6.25 | 0.563 | 3.13 | 0.75 |
| Octyl gallate | 12.5 | 0.252 | 3.13 | 0.75 |
| Nonyl gallate | 6.25 | 0.625 | 12.5 | 0.502 |
| Decyl gallate | 12.5 | 0.258 | 6.25 | 0.508 |
| Undecyl gallate | 12.5 | 0.313 | 6.25 | 0.251 |
| Dodecyl gallate | 25 | 0.531 | 50 | 0.375 |

### Result

As shown in Table 5, the gallates were found to have the antibacterial activity and the effects of the combination use with oxacillin in the tested MRSA, i.e, COL Strain and Strain No.5. In Table 5, MIC means those of the various gallates themselves and the antibacterial activities of octyl gallate, nonyl gallate, decyl gallate, and undecyl gallate were most potent when used alone. On the other hand, butyl gallate and isobutyl gallate were found to have the most potent effect of the combination use by referring to their FIC values, which show the effect of the combination use with oxacillin.

### Examples

### Example 1 Tablet

According to the conventional process, 50mg of isoamyl gallate, 50mg of oxacillin, 1 g of lactose, 300 mg of starch, 50 mg of methylcellulose and 30 mg of talc are formulated to give ten tablets which are then coated with sucrose.

### Example 2-17 Tablet

According to the example 1 wherein isoamyl gallate is replaced with methyl gallate, ethyl gallate, n-propyl gallate, n-butyl gallate, n-pentyl gallate, n-hexyl gallate, n-heptyl gallate, n-octyl gallate, n-nonyl gallate, n-decyl gallate, n-undecyl gallate, n-lauryl gallate or isobutyl gallate tablets are prepared.

### Example 18 Injectable

A sterile mixture containing 500 mg of methyl gallate and 500 mg of oxacillin are placed in a sterilized vial which is then sealed. Upon use this mixture is dissolved in saline to give an injectable.

### Example 19-26 Injectable

According to example 18, wherein methyl gallate is replaced with ethyl gallate, n-propyl gallate, n-butyl gallate, n-pentyl gallate or isobutyl gallate an injectable is prepared.

### Example 27 Disinfectant .

A disinfectant prepared from isoamyl gallate, oxacillin, ethanol, and sterilized water is shown. These ingredients are mixed with the ratio below; isoamyl gallate 25 mg oxacillin 10 mg ethanol 500 ml sterilized water 500 ml

### Industrial applicability

The phenol derivatives of formula V' can potentiate the antibacterial effect of β-lactam antibiotics when used in the combination with these antibiotics. Furthermore, they can reduce the amount of β-lactams clinically used, and chances for the bacteria to acquire resistance to β-lactam antibiotics in advance. Accordingly, the invention is used as a pharmaceutical composition for the treatment of infections with MRSA utilizing the characteristic that the phenol derivatives of formula V' potentiate the antibacterial effect of β-lactam antibiotics; the invention is also used as a disinfectant or functional food comprising the phenol derivatives of formula V', which has antibacterial activity against MRSA.

## Claims

1. A phenol derivative of formula V'; 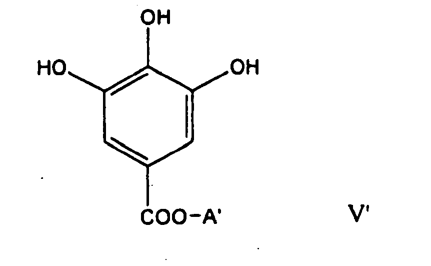 wherein A' is a lower alkyl group having 1 to 12 carbon atoms, or a pharmaceutically acceptable salt thereof, for use as an enhancer of β-lactam antibiotics for the treatment of MRSA infections.

2. The phenol derivative of claim 1 wherein the lower alkyl group is selected from the group of methyl, ethyl, n-propyl, n-butyl, isobutyl, n-pentyl, isoamyl, hexyl, heptyl, octyl, nonyl, decyl, undecyl and dodecyl.

3. The phenol derivative of claim 1 or 2 wherein the enhancer is used for the treatment of MRSA infections in combination with a β-lactam antibiotic.

4. The phenol derivative of claim1 or 2 wherein the enhancer is used in a disinfectant for MRSA in combination with a β-lactam antibiotic.

5. The phenol derivative of claim 1 or 2 wherein the enhancer is used in a functional food having antibacterial activity for MRSA in combination with a β-lactam antibiotic.

6. Use of a phenol derivative of formula V'; 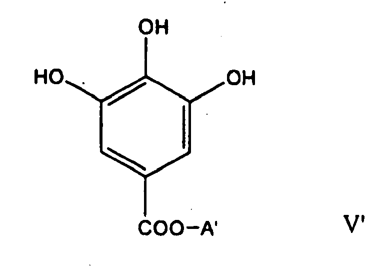 wherein A' is a lower alkyl group having 1 to 12 carbon atoms, or a pharmaceutically acceptable salt thereof, in the manufacture of an enhancer of β-lactam antibiotics for the treatment of MRSA infections.

7. A pharmaceutical composition for a therapy of MRSA infections comprising a β-lactam antibiotic and a phenol derivative of formula V' as defined in claim 1 or 2, or a pharmaceutically acceptable salt thereof.

8. A disinfectant for MRSA comprising a β-lactam antibiotic and a phenol derivative of formula V' as defined in claim 1 or 2, or a pharmaceutically acceptable salt thereof.

9. A functional food for a therapy of MRSA infections comprising a β-lactam antibiotic and a phenol derivative of formula V' as defined in claim 1 or 2, or a pharmaceutically acceptable salt thereof.

## Patentansprüche

1. Phenolderivat der Formel (V'): 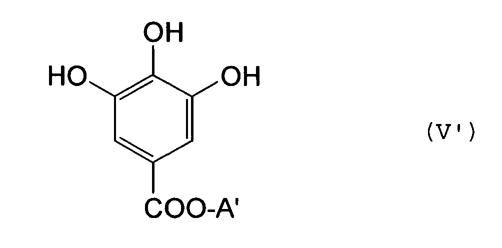 worin A' eine niedere Alkylgruppe mit 1 bis 12 Kohlenstoffatomen ist, oder ein pharmazeutisch annehmbares Salz davon zur Verwendung als Verstärker von β-Lactam-Antibiotika für die Behandlung von MRSA-Infektionen.

2. Phenolderivat gemäß Anspruch 1, worin die niedere Alkylgruppe ausgewählt ist aus der Gruppe von Methyl, Ethyl, n-Propyl, n-Butyl, Isobutyl, n-Pentyl, Isoamyl, Hexyl, Heptyl, Octyl, Nonyl, Decyl, Undecyl und Dodecyl.

3. Phenolderivat gemäß Anspruch 1 oder 2, worin der Verstärker für die Behandlung von MRSA-Infektionen zusammen mit einem β-Lactam-Antibiotikum verwendet wird.

4. Phenolderivat gemäß Anspruch 1 oder 2, worin der Verstärker in einem Desinfektionsmittel für MRSA zusammen mit einem β-Lactam-Antibiotikum verwendet wird.

5. Phenolderivat gemäß Anspruch 1 oder 2, worin der Verstärker in einem funktionellen Lebensmittel mit antibakterieller Aktivität für MRSA zusammen mit einem β-Lactam-Antibiotikum verwendet wird.

6. Verwendung eines Phenolderivats der Formel (V'): 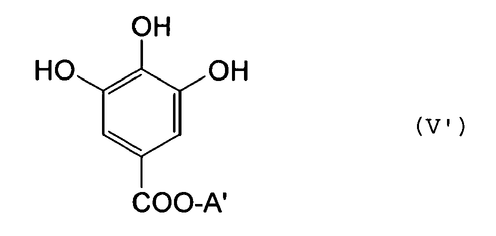 worin A' eine niedere Alkylgruppe mit 1 bis 12 Kohlenstoffatomen ist, oder eines pharmazeutisch annehmbaren Salzes davon in der Herstellung eines Verstärkers von β-Lactam-Antibiotika für die Behandlung von MRSA-Infektionen.

7. Pharmazeutische Zusammensetzung für eine Therapie von MRSA-Infektionen, die ein β-Lactam-Antibiotikum und ein wie in Anspruch 1 oder 2 definiertes Phenolderivat der Formel (V') oder ein pharmazeutisch annehmbares Salz davon umfaßt.

8. Desinfektionsmittel für MRSA, das ein β-Lactam-Antibiotikum und ein wie in Anspruch 1 oder 2 definiertes Phenolderivat der Formel (V') oder ein pharmazeutisch annehmbares Salz davon umfaßt.

9. Funktionelles Lebensmittel für eine Therapie von MRSA-Infektionen, das ein β-Lactam-Antibiotikum und ein wie in Anspruch 1 oder 2 definiertes Phenolderivat der Formel (V') oder ein pharmazeutisch annehmbares Salz davon umfaßt.

## Revendications

1. Dérivé de phénol de formule V' : 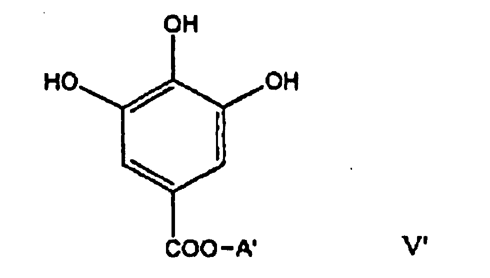 dans laquelle A' est un groupe alkyle inférieur comportant de 1 à 12 atomes de carbone, ou un sel pharmaceutiquement acceptable de celui-ci, pour une utilisation en tant qu'amplificateur de β-lactamines pour le traitement d'infections par le MRSA (staphylocoque doré résistant à la méticiline).

2. Dérivé de phénol selon la revendication 1, dans lequel le groupe alkyle inférieur est choisi parmi le groupe de méthyle, éthyle, n-propyle, n-butyle, isobutyle, n-pentyle, isoamyle, hexyle, heptyle, octyle, nonyle, décyle, undécyle et dodécyle.

3. Dérivé de phénol selon la revendication 1 ou 2, dans lequel l'amplificateur est utilisé pour le traitement d'infections par le MRSA en combinaison avec une β-lactamine.

4. Dérivé de phénol selon la revendication 1 ou 2, dans lequel l'amplificateur est utilisé dans un désinfectant pour le MRSA en combinaison avec une β-lactamine.

5. Dérivé de phénol selon la revendication 1 ou 2, dans lequel l'amplificateur est utilisé dans un aliment fonctionnel ayant une activité anti-bactérienne pour le MRSA en combinaison avec une β-lactamine.

6. Utilisation d'un dérivé de phénol de formule V': 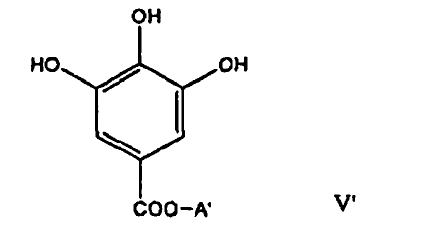 dans laquelle A' est un groupe alkyle inférieur comportant de 1 à 12 atomes de carbone, ou un sel pharmaceutiquement acceptable de celui-ci, dans la fabrication d'un amplificateur de β-lactamines pour le traitement d'infections par le MRSA.

7. Composition pharmaceutique pour une thérapie d'infections par le MRSA comprenant une β-lactamine et un dérivé de phénol de formule V' comme défini dans la revendication 1 ou 2, ou un sel pharmaceutiquement acceptable de celui-ci.

8. Désinfectant pour le MRSA comprenant une β-lactamine et un dérivé de phénol de formule V' comme défini dans la revendication 1 ou 2, ou un sel pharmaceutiquement acceptable de celui-ci.

9. Aliment fonctionnel pour la thérapie d'infections par le MSRA comprenant une β-lactamine et un dérivé de phénol de formule V' comme défini dans la revendication 1 ou 2, ou un sel pharmaceutiquement acceptable de celui-ci.
